# EUROPEAN PATENT SPECIFICATION

(11) **EP 1 276 495 B1**
(45) Date of publication and mention of the grant of the patent: **19.04.2006**
(21) Application number: 01943250.9
(22) Date of filing: 12.04.2001
(51) Int. Cl.: A61K 38/17, A61P 11/00

(54) **USE OF RECOMBINANT PULMONARY SURFACTANT FOR THE EARLY TREATMENT OF ACUTE PULMONARY DISEASES**
VERWENDUNG VON REKOMBINANTEM LUNGENSURFACTANT ZUR FRÜHBEHANDLUNG VON AKUTEN LUNGENERKRANKUNGEN
UTILISATION DE TENSIOACTIF PULMONAIRE RECOMBINANT POUR LE TRAITEMENT PRECOCE DE TROUBLES PULMONAIRES AIGUS

(30) Priority: 12.04.2000 EP 00107858
(43) Date of publication of application: 22.01.2003
(73) Proprietor: ALTANA Pharma AG, 78467 Konstanz (DE)
(72) Inventor: HÄFNER, Dietrich, 78464 Konstanz (DE); KELLER, Andreas, 78479 Reichenau (DE); RATHGEB, Frank, 78465 Konstanz (DE); SCHAFFER, Peter, 78315 Radolfzell (DE); WURST, Wilhelm, 78465 Konstanz (DE); KARL, Christoph, 3002 Purkersdorf (AT)
(86) International application number: PCT/EP2001/004223
(87) International publication number: WO 2001/076619

(56) References cited:
- WO-A-00/27360
- WO-A-00/43026
- WO-A-92/04907
- WO-A-98/49191
- DE-A- 19 827 907
- US-A- 5 874 406
- HAEFNER D ET AL: "EFFECTS OF EARLY TREATMENT WITH RSP-C SURFACTANT ON OXYGENATION ANDHISTOLOGY IN RATS WITH ACUTE LUNG INJURY" PULMONARY PHARMACOLOGY AND THERAPEUTICS,ACADEMIC PRESS, NEW YORK, NY,US, vol. 12, no. 3, 1999, pages 193-201, XP000971638 ISSN: 1094-5539
- LIU M ET AL: "Pulmonary surfactant given prophylactically alleviates an asthma attack in guinea-pigs" CLINICAL AND EXPERIMENTAL ALLERGY, vol. 26, no. 3, 1996, pages 270-275, XP000952897
- GRIESE M: "Pulmonary surfactant in health and human lung diseases: State of the art." EUROPEAN RESPIRATORY JOURNAL, vol. 13, no. 6, June 1999 (1999-06), pages 1455-1476, XP000971645 ISSN: 0903-1936
- HAEFNER DIETRICH ET AL: "ARDS model in the rat: Influence of early and late treatment with surfactant in an animal model of acute lung injury." ARZNEIMITTEL-FORSCHUNG, vol. 48, no. 3, March 1998 (1998-03), pages 318-320, XP002173655 ISSN: 0004-4172

## Description

### Technical field of the invention

The invention relates to the novel use of recombinant pulmonary surfactant preparations for the early treatment of acute pulmonary diseases.

### Prior art

ARDS (Adult Respiratory Distress Syndrome) is a descriptive expression which is applied to a large number of acute, diffuse infiltrative pulmonary lesions of differing etiology if they are associated with a severe gas exchange disorder (in particular arterial hypoxemia). (G.R. Bernard et at.: Report of the American-European consensus conference on ARDS: definitions, mechanisms, relevant outcomes and clinical trial coordination; Intensive Care Medicine, 1994, 20:225-232). The expression ARDS is used for IRDS (Infant Respiratory Distress Syndrome) because of the numerous common clinical and pathological features. If, in the case of IRDS, the lung surfactant deficiency caused by premature birth is predominant, then in the case of ARDS a lung surfactant malfunction is caused by the disease of the lung based on differing etiologies. Triggering causes for an ALI (Acute Lung Injury) including ARDS can, for example, be (cited in accordance with Harrison's Principles of Internal Medicine 10th Ed. 1983 McGraw-Hill lnt. Book Comp.) diffuse pulmonary infections (e.g. due to viruses, bacteria, fungi), aspiration of, for example, gastric juice or in the case of near-drowning, inhalation of toxins or irritants (e.g. chlorine gas, nitrogen oxides, smoke), direct or indirect trauma (e.g. multiple fractures or pulmonary contusion), systemic reactions to inflammations outside the lung (e.g. hemorrhagic pancreatitis, gram-negative septicemia), transfusions of high blood volumes or alternatively after cardiopulmonary bypass.

The therapy of ARDS present mainly consists in the earliest possible application of different forms of ventilation [e.g. PEEP (positive end-expiratory pressure), raising of the oxygen concentration of the respiratory air, SIMV (Synchronized Intermittent Mandatory Ventilation; Harrison's Principles of Internal Medicine 10th Ed. 1983 McGraw-Hill lnt. Book Comp)) up to extracorporeal membrane oxygenation (ECMO; Zapol and Lemaire Adult Respiratory Distress Syndrome, Marcel Dekker Inc. 1991). The specific use of various ventilation techniques has only led to a small lowering of mortality and includes the risk of setting in motion a vicious circle. By ventilation with pressure and high FiO₂ (Fraction of Inspired Oxygen; proportion of oxygen in the respiratory air), the lungs themselves can be damaged and as a result of this even higher pressures and higher FiO₂ may be required in order to obtain an adequate oxygenation of the blood. For many years, it has proven suitable to treat IRDS by introducing pulmonary surfactant preparations into the lungs of the children concerned. It is known from pilot studies that pulmonary surfactant preparations are also clinically active in ALI including ARDS (survey, for example, B. Lachmann, D. Gommers and E. P. Eijking: Exogenous surfactant therapy in adults, Atemw.-Lungenkrkh. 1993, 19:581-91; D. Walmrath et al.: Bronchoscopic surfactant administration in patients with severe adult respiratory distress syndrome and sepsis, Am. J. Respir. Crit. Care Med. 1996, 154:57-62; T. J. Gregory et al.: Bovine surfactant therapy for patients with acute respiratory distress syndrome, Am. J. Respir. Crit. Care Med. 1997, 155:1309-15).

Surfactant abnormatities of differing severity are also reported for a number of other disease conditions, for example in obstructive pulmonary disorders such as asthma, bronchiolitis, COPD (Chronic Obstructive Pulmonary Disease) and after lung transplantation or alternatively after cardiopulmonary bypass (survey, see, for example, M. Griese Eur. Respir. J. 1999; 13: 1455-1467). Macnaughton et al. (Chest 1994; 105: 421-425) and DoCampo et al. (Lancet 1994; 343: 482) describe the administration of exogenous surfactant after cardiopulmonary bypass. McBrien et al. (Lancet 1993; 342:1485-1486) and Suzuki et al. (Eur. J. Pediatr. 1996; 155: 383-384) describe the administration of surfactant after near-drowning. Struber et al. (Cardiovasc. Surg. 1995; 110: 563-564) describe the administration of surfactant after lung transplantation.

### Description of the invention

The object of the present invention is the provision of medicaments for the early treatment of acute pulmonary diseases. Surprisingly, it has now been found that pulmonary surfactant preparations which contain recombinant surfactant proteins, are suitable for the early treatment of acute pulmonary diseases in humans. Thus, for example, by the treatment of ALI or ARDS patients with recombinant pulmonary surfactant preparations the intensity of ALI or ARDS can be attenuated and thus the mortality rate associated with ALI or ARDS can be lowered. In particular, a progression to ARDS can be prevented even in patients with ALI or the intensity of ARDS can be attenuated. The stay of patients in intensive care units can be shortened and thus costs can be saved. Furthermore, in patients who are ventilated, it is possible by the administration of recombinant pulmonary surfactant to avoid side effects of ventilation, for example the risk of a nosocomial infection or pneumonia for the patients can be lowered or prevented.

In a first aspect, the invention therefore relates to the use of a pulmonary surfactant preparation comprising phospholipids and lusupultide for the production of medicaments for the early treatment of acute pulmonary diseases in humans.

Exemplary acute lung diseases according to the invention are ALI, ARDS, acute respiratory insufficiency, pneumonias (in particular ventilation-induced pneumonias), nosocomial infections or SIRS (systemic inflammatory response syndrome) associated with ALI.

According to the invention, humans are preferably patients in which the risk of the development of an acute lung disease exists. In particular, these are patients at risk of ALI or ARDS, patients in which the risk of acute respiratory insufficiency exists, patients in which the risk of pneumonia exists, patients in which the risk of a nosocomial infection exists, patients with hypothermia or patients with SIRS associated with ALI. By way of example, patients selected from the following patient groups may be mentioned: patients before, during or after intervention on the open thorax, patients who are ventilated, patients with pulmonary intoxication, patients with trauma, patients with sepsis, patients with pneumonia or those in which the risk of pneumonia exists, patients with a nosocomial infection or in which the risk of a nosocomial infection exists, patients with hypothermia and patients with SIRS associated with ALI.

According to the invention, early treatment of acute lung diseases in mammals is understood as meaning the treatment of mammals which are in the stage of development of an acute lung disease.

Natural pulmonary surfactant has surface-active properties; it reduces, for example, the surface tension in the alveoli. A simple and rapid in vitro test with which the surface activity of pulmonary surfactant can be determined is, for example, the so-called Wilhelmy balance [Goerke, J. Biochim. Biophys. Acta, 344: 241-261 (1974), King R.J. and Clements J.A., Am. J. Physicol. 223: 715-726 (1972)]. This method gives information on the pulmonary surfactant quality, measured as the action of a pulmonary surfactant of achieving a surface tension of almost zero mN/m. Another measuring device for determining the surface activity of pulmonary surfactant is the pulsating bubble surfactometer [Possmayer F., Yu S. and Weber M., Prog. Resp. Res., Ed. v. Wichert, Vol. 18: 112-120 (1984)].

The activity of a pulmonary surfactant preparation can also be determined by means of in vivo tests, for example as described by Hafner et al. (D. Häfner et al.: Effects of rSP-C surfactant on oxygenation and histology in a rat lung lavage model of acute lung injury. Am. J. Respir. Crit. Care Med. 1998, 158: 270-278). By the measurement of, for example, the pulmonary compliance, the blood gas exchange or the ventilation pressures needed, it is possible to obtain information on the activity of a pulmonary surfactant.

Pulmonary surfactant preparation is understood according to the invention as comprising phospholipids and the recombinant SP-C derivative which is disclosed in WO 95/32992, and which differs from human SP-C in positions 4 and 5 by the replacement of cysteine by phenylalanine and in position 32 by the replacement of methionine by isoleucine [designated below as rSP-C (FF/I) or lusupultide (INN)].

As further constituents which can be present in pulmonary surfactant preparations, fatty acids such as palmitic acid may be mentioned. The pulmonary surfactant preparations can also contain electrolytes such as calcium, magnesium and/or sodium salts (for example calcium chloride, sodium chloride and/or sodium hydrogencarbonate) in order to establish an advantageous viscosity. Preferred preparations according to the invention contain 80 to 95% by weight of phospholipids, 0.5 to 3.0% by weight of pulmonary surfactant proteins, 3 to 15% by weight of fatty acid, preferably palmitic acid, and 0 to 3% by weight of calcium chloride.

The pulmonary surfactant preparations are prepared by processes known per se and familiar to the person skilled in the art, for example as described in WO 95/32992. According to the invention, the pulmonary surfactant preparations are preferably lyophilized and in particular spray-dried pulmonary surfactant preparations. Lyophilized preparations are disclosed, for example, in WO 97/35882, WO 91/00871 and DE 3229179. WO 97/26863 describes a process for the preparation of powdered pulmonary surfactant preparations by spray drying. According to the invention, preparations prepared in this way are preferred.

The patients having interventions on the open thorax can be, according to the invention, patients in which an intervention is carried out on the heart, such as a bypass operation or a heart valve operation. Furthermore, they can be patients in which an intervention is carried out on the lung, such as a lung transplantation or a pneumonectomy. In connection with the treatment of patients with lung transplantations, according to the invention a preliminary treatment of the organ to be transplanted with pulmonary surfactant preparation is preferably carried out before the transplantation, in particular before the storage of the transplant, particularly preferably before removal of the transplant from the organ donor. Novick et al. (Evaluation of Surfactant Treatment Strategies after Prolonged Graft Storage in Lung Transplantation; Am. J. Respir. Crit. Care Med. 1996, Vol. 154, 98-104) describe surfactant treatment strategies in connection with storage of lung transplants. Preferably, the patient who receives the organ donation is pretreated before transplantation.with pulmonary surfactant preparation. After transplantation has taken place, further treatment of the patient with the pulmonary surfactant preparation can then be carried out.

According to the invention, ventilation of a patient is understood as meaning ventilation of the lungs which is brought about by aids or artificial respiration. An exemplary aid for ventilation which may be mentioned is the respirator, where different forms of ventilation known to the person skilled in the art can be used. Patients who are ventilated are, in particular, patients where spontaneous respiration is absent or insufficient. By way of example, patients having respiratory insufficiency, patients having central or peripheral respiratory paralysis, patients having ventilation under anesthesia, patients having long-term ventilation in intensive medicine and patients who are ventilated in the course of resuscitation. The ventilation of patients contains the risk of damaging the lung (Ventilation-Induced Lung Injury; VILI) and setting a vicious circle in motion. As a result of ventilation using pressure and a high FiO₂ (Fraction of Inspired Oxygen; proportion of oxygen in the respiratory air), the lung itself can be damaged and this can have the result that even higher pressures and higher FiO₂ are needed in order to obtain adequate oxygenation of the blood. At the same time, in the case of mechanically ventilated patients an increased risk of pneumonias and nosocomial infections exists (Michael J. Richards et al. Critical Care Medicine 1999; 27:887-892).

Patients having pulmonary intoxication can be, for example, patients having pulmonary intoxication as a result of a bone marrow transplantation (toxic lung injury after bone marrow transplantation), or patients having a pulmonary intoxication which was caused by toxic gases.

The patients having trauma are, according to the invention, in particular patients having thoracic, cranial or cerebral trauma or those having multiple traumas.

Patients having hypothermia are in particular patients having a lowered body temperature in the case of collapse, hypothyroidism, cachexia, accidental hypothermia due to exposure to cold (e.g. in mountain and drowning accidents) and controlled hypothermia as is used, for example, in open heart surgery, in neurosurgery and in transplantations.

The pulmonary surfactant preparation is to be administered in a manner known to the person skilled in the art, preferably by intratracheal instillation (infusion or bolus) of a pulmonary surfactant solution or suspension or in the form of an atomization of a pulmonary surfactant solution or suspension or by atomization of pulmonary surfactant powder. Preferably, the preparations according to the invention for administration are dissolved or suspended in a suitable solvent or resuspension medium, in particular if the preparations are present in lyophilized or spray-dried form. Preferably, the suitable resuspension medium is a physiological saline solution. It has proven advantageous to administer suspensions or solutions of the preparations according to the invention which contain 12.5 to 100 mg of phospholipids per ml of suspension. Preferably, the preparations according to the invention are administered per application in such an amount that the amount of phospholipids is between 12.5 and 200 mg per kilogram of body weight. As a rule, administration is carried out 1 to 3 times daily over a period of 1 to 7 days. A process is preferred in which the pulmonary surfactant solution employed contains 0.5 to 2.0 mg of rSP-C (FF/I) per ml of solvent. Particular mention may be made of a process in which the pulmonary surfactant solution employed contains 0.75 to 1.5 mg of rSP-C (FF/t) per ml of solvent. If desired, before the administration of the preparations according to the invention a bronchoalveolar lavage, preferably with dilute pulmonary surfactant preparation, can be carried out. Such a procedure is described, for example, in Gommers et al. [Bronchoalveolar lavage with a diluted surfactant suspension prior to surfactant instillation improves the effectiveness of surfactant therapy in experimental acute respiratory distress syndrome (ARDS), Intensive Care Med. 1998, 24:494-500] and in WO 98/49191.

If desired, the pulmonary surfactant preparations can also be administered in combination with other medicaments in the early treatment of acute lung diseases, in particular with those medicaments which are customarily employed for the treatment of acute lung diseases.

### Examples

### A.) Production of powdered pulmonary surfactant preparations

Powdered pulmonary surfactant preparations are produced by the process described in WO 97/26863:

### Example 1

7.0 g of 1,2-dipalmitoyl-3-sn-phosphatidylcholine, 2.5 g of 1-palmitoyl-2-oleoyl-3-sn-phosphatidylglycerol sodium, 205 mg of calcium chloride dihydrate and 250 mg of palmitic acid are dissolved in 300 ml of ethanol/water (85:15) with warming to 60°C, cooled to room temperature and mixed with 350 ml of a solution of rSP-C (FF/I) in chloroform/methanol 9:1 (c = 429 mg/l). The resulting solution is spray-dried in a Buchi B 191 laboratory spray dryer. Spray conditions: drying gas air, inlet temperature 90°C, outlet temperature 52 - 54°C. A relatively loose powder is obtained.

### Example 2 (illustrative, not part of the invention)

A solution of the surfactant obtained from bovine lungs (obtained by extraction and purification steps such as described, for example, in EP 406732) in chloroform/methanol is spray-dried under the following conditions: Büchi B 191 laboratory spray dryer, drying gas nitrogen, inlet temperature 80°C, outlet temperature 50 - 52°C. A fine, yellowish powder is obtained.

### Example 3

10.95 g of 1,2-dipalmitoyl-3-sn-phosphatidylcholine, 4.6 g of 1-palmitoyl-2-oleoyl-3-sn-phosphatidylglycerol ammonium, 418 mg of calcium chloride dihydrate and 750 mg of palmitic acid are dissolved in 330 ml of 2-propanol/water (85:15) at 50°C and, after cooling to 30°C, mixed with 620 ml of a solution of rSP-C (FF/I) in isopropanol/water (95:5, c = 484 mg/l). The resulting solution is spray-dried in a Buchi B 191 laboratory spray dryer. Spray conditions: drying gas nitrogen, inlet temperature 100°C, outlet temperature 58 - 60°C. A colorless powder is obtained.

### Example 4

3.74 g (5.1 mmol) of 1,2-dipalmitoyl-3-sn-phosphatidylcholine, 2.81 g (3.7 mmol) of 1-palmitoyl-2-oleoyl-3-sn-phosphatidylcholine, 2.90 g (3.9 mmol) of 1,2-dipalmitoylphosphatidyt-3-sn-phosphatidylglycerol sodium. 234 mg of palmitic acid and 279 mg (1.9 mmol) of calcium chloride dihydrate are dissolved in 160 ml of 2-propanol/water (85 : 15) at 50°C and, after cooling to 30°C, mixed with 566 ml of a solution of rSP-C (FF/l) in isopropanol/water (92 : 8, c = 330 mg/l) at 30°C. The resulting solution is spray-dried in a Büchi B 191 laboratory spray dryer. Spray conditions: drying gas nitrogen, inlet temperature 90°C, outlet temperature 58 - 60°C. A colorless powder is obtained.

### Example 5 (illustrative, not part of the invention)

0.5 g of KL4 (INN: sinapultide), 7.125 g of 1,2-dipalmitoyl-3-sn-phosphatidylchofine and 2.43 g of 1-palmitoyl-2-oleoyl-3-sn-phosphatidylglycerol ammonium are dissolved in 500 ml of chloroform/methanol 1 : 1 with warming to 45°C and then spray-dried in a Buchi B 191 laboratory spray dryer. Spray conditions: drying gas nitrogen, inlet temperature 85°C, outlet temperature 55°C. A colorless powder is obtained.

### Example 6

A solution of phospholipids, palmitic acid and calcium chloride dihydrate obtainable according to Example 1, 3 or 4 is spray-dried - without addition of a solution of rSP-C (FF/I) - corresponding to the conditions according to Example 1, 3 or 4. A powder is obtained.

### B.) Production of the medicaments according to the invention

### Example 1

0.1 to 10 g of the powder obtained according to Example 1 are dispensed into a bottle of volume 100 to 250 ml and the bottle is sealed. The bottle is packed in a suitable folding box together with a pack insert.

## Claims

1. The use of a pulmonary surfactant preparation, comprising phospholipids and lusupultide for the production of medicaments for the early treatment of acute pulmonary diseases In humans.

2. The use as daimed In claim 1, the pulmonary surfactant preparation contains 80 to 95 % by weight of phasphotipids, 0,5 to 3,0 % by weight of pulmonary surfactant proteins, 3 to 15 % by weight of fatty acids, and 0 to 3 % by weight of calcium chloride.

3. The use as claimed in claim 1, the humans being patients in which the risk of ARDS or ALI exists, patients in which the risk of acute respiratory insufficiency exists, patients in which the risk of pneumonia exists, patients in which the risk of a nosocomial infection exists, patients with hypothermia or patients with SIRS (systemic Inflammatory response syndrome) associated with ALI.

4. The use as claimed In claim 3, patients being selected from the following patient groups: patients before, during or after an intervention on the open thorax, patients who are ventilated, patients with pulmonary intoxication, patients with a trauma, patients with sepsis, patients with pneumonia or those in which the risk of pneumonia exists, patients with a nosocomial infection or in which the risk of a nosocomial infection exists, patients with hypothermia and patients with SIRS (systemic Inflammatory response syndrome) associated with ALI.

## Revendications

1. Utilisation d'une préparation tensioactive pulmonaire, comprenant des phospholipides et du lusupultide pour la production de médicaments destinés au traitement précoce de maladies pulmonaires aiguë chez des êtres humains.

2. Utilisation selon la revendication 1, la préparation tensioactive pulmonaire contenant de 80 à 95 % en poids de phospholipides, de 0,5 à 3,0 % en poids de protéines tensioactives pulmonaires, de 3 à 15 % en poids d'acides gras, et de 0 à 3 % en poids de chlorure de calcium.

3. Utilisation selon la revendication 1, les êtres humains étant des patients chez lesquels il existe un risque de SDRA ou de ALI, des patients chez lesquels il existe un risque d'insuffisance respiratoire aiguë, des patients chez lesquels il existe un risque de pneumonie, des patients chez lesquels il existe un risque d'une infection nosocomiale, des patients présentant une hypothermie ou des patients présentant un SIRS (syndrome de réponse inflammatoire systémique) associé à un ALI.

4. Utilisation selon la revendication 3, les patients étant choisis parmi les groupes de patients suivants : des patients avant, durant ou après une intervention sur le thorax ouvert, des patients qui sont sous ventilation, des patients présentant une intoxication pulmonaire, des patients présentant un traumatisme, des patients présentant une septicémie, des patients présentant une pneumonie ou ceux chez lesquels il existe un risque de pneumonie, des patients présentant une infection nosocomiale ou chez lesquels il existe un risque d'une infection nosocomiale, des patients présentant une hypothermie et des patients présentant un SIRS (syndrome de réponse inflammatoire systémique) associé à un ALI.

## Patentansprüche

1. Verwendung einer Lungensurfactant-Zubereitung, die Phospholipide und Lusupultid enthält, zur Herstellung von Arzneimitteln zur Frühbehandlung von akuten Lungenerkrankungen in Menschen.

2. Verwendung nach Anspruch 1, wobei die Lungensurfactant-Zubereitung 80 bis 95 Gew.-% Phospholipide, 0,5 bis 3,0 Gew.-% Lungensurfactant-Proteine, 3 bis 15 Gew.-% Fettsäuren und 0 bis 3 Gew.-% Calciumchlorid enthält.

3. Verwendung nach Anspruch 1, wobei es sich bei den Menschen um Patienten handelt, bei denen das Risiko von ARDS oder ALI besteht, um Patienten, bei denen das Risiko einer akuten respiratorischen Insuffizienz besteht, um Patienten, bei denen das Risiko einer Pneumonie besteht, um Patienten, bei denen das Risiko einer Nosocomialinfektion besteht, um Patienten mit Hypothermie oder um Patienten mit SIRS (Systemic Inflammatory Response Syndrome) assoziiert mit ALI.

4. Verwendung nach Anspruch 3, wobei es sich um Patienten ausgewählt aus folgenden Patientengruppen handelt: Patienten vor, während bzw. nach einem Eingriff am offenen Thorax, Patienten, die beatmet werden, Patienten mit Lungenintoxikation, Patienten mit einem Trauma, Patienten mit Sepsis, Patienten mit Pneumonie bzw. solche, bei denen das Risiko einer Pneumonie besteht, Patienten mit einer Nosocomialinfektion oder bei denen das Risiko einer Nosocomialinfektion besteht, Patienten mit Hypothermie und Patienten mit SIRS (Systemic Inflammatory Response Syndrome) assoziiert mit ALI.
